# EUROPEAN PATENT APPLICATION

(11) **EP 4 299 061 A2**
(43) Date of publication of application: **03.01.2024**
(21) Application number: 23209796.4
(22) Date of filing: 18.04.2016
(51) Int. Cl.: A61K 31/155

(54) **FASTING MIMICKING DIET (FMD) AND GLUCOSE LOWERING DRUGS PROTECT NORMAL CELLS AND GENERATE CANCER SENSITIZING CONDITIONS IN RESPONSE TO STANDARD AND HIGH GLUCOSE CONDITIONS INDUCED BY RAPAMYCIN AND DEXAMETHASONE**

(30) Priority: 16.04.2015 US 201562148451 P
(62) Divisional of application: 16780985.4
(71) Applicant: University of Southern California, Los Angeles, CA 90015 (US)
(72) Inventor: LONGO, Valter D., Playa del Rey, 90293 (US); DI BIASE, Stefano, LOS ANGELES, 90015 (US)
(74) Representative: GPI Brevets

(57) **Abstract**

A method comprising administrating a fasting mimicking diet for a first time period and administrating metformin during the re-feeding period.

## Description

This application claims the benefit of U.S. provisional application Serial No. 62/148,451 filed April 16, 2015, the disclosure of which is hereby incorporated in its entirety by reference herein.

### STATEMENT REGARDING FEDERALLY SPONSORED RESEARCH OR DEVELOPMENT

The invention was made with Government support under Contract No. 1PO1AG034906 awarded by the National Institutes of Health. The Government has certain rights to the invention.

### TECHNICAL FIELD

In at least one aspect, the present invention relates to methods to protect normal tissues from increased toxicity/sensitization to chemotherapy drugs induced by the raised circulating glucose levels upon administration of rapamycin and the palliative drug dexamethasone.

### BACKGROUND

Cancer management and treatment has significantly improved over the past century. However, the standard of care is still predominantly uses chemotherapy, radiotherapy or their combination. Both modes of treatment are associated with a multitude of side effects ranging from discomfort to development of secondary cancers and organ toxicity especially heart and liver. To increase efficacy of cancer treatment and to help with management of symptoms, other drugs such as dexamethasone are often used in combination with chemo- and radio- therapy. Dexamethasone (Dexa), commonly combined with chemotherapy, is often used as a palliative drug that has also been shown to be effective in treating multiple myeloma, leukemia, and lymphoma. However, treatment with Dexa can be causative of a number of side effects including, fluid retention, weight gain, heartburn, insomnia and elevated levels of blood glucose.

It has previously been shown that Short-Term Starvation (STS) is an effective practice to ease the discomfort associated with cancer treatment while increasing the efficacy of such treatments. Moreover, it has been demonstrated that STS regimens are an effective method in protecting normal cells and tissues during chemotherapy (Differential Stress Resistance or DSR) (Raffaghello et.al., PNAS. 2008; PMID: 18378900, and Lee et.al. Cancer Research. 2010; PMID: 20145127).

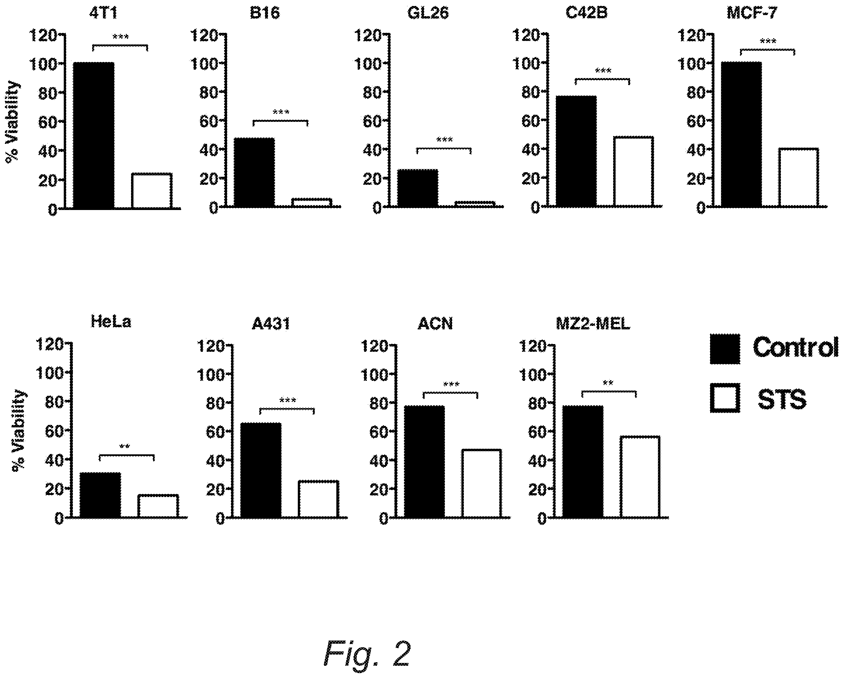

It has been previously shown that fasting can sensitize cancer cells but not normal cells to chemotherapy, a phenomena referred to as Differential Stress Sensitization (DSS), which efficacy has been attributed to the reduction in circulating glucose and IGF-1 levels (**Figure 2**) (Lee et.al. Sci Transl Med. 2012; PMID: 22323820). However, a 10- to 14-day re-feeding period between fasting cycles is needed to recover the body weight loss.

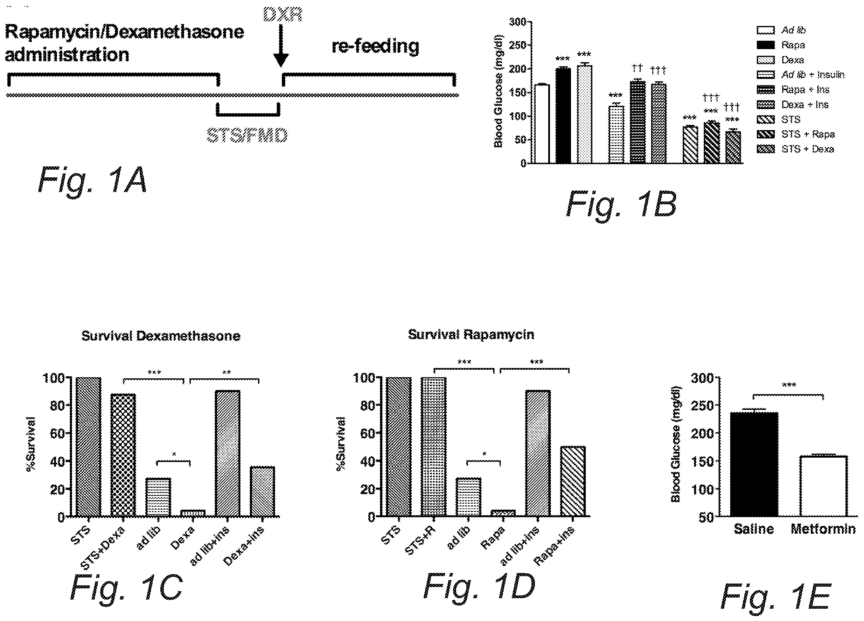

5' AMP-activated protein kinase (AMPK) is an enzyme up-regulated during STS/FMD regimen and which plays a role in cellular energy homeostasis and has been associated with lifespan extension. AMPK is also considered a metabolic tumor suppressor (Luo et.al. Future Oncol. 2010; PMID: 20222801). Metformin, is an AMPK activator that leads to the reduction of circulating glucose (**Figure 1E**) and has potential for the treatment/prevention of cancer.

The central players that regulate metabolism in all living cells do so by modulating normal-cell growth in part by regulating serine/threonine protein kinases, which has led to the modified standard of care that included administration of kinases inhibitors such as rapamycin (Rapa) in combination with chemotherapy. Kinases and other signal transduction inhibitors can delay cancer growth and are widely used but, like dexamethasone, can also cause major side effects to normal cells.

Accordingly, there is a need for treatment protocols that (i) mitigate the side effects associated with the adjunct drugs used in chemotherapy, and (ii) can maintain reduced glucose levels during the "re-feeding" period between STS cycles or to substitute STS/FMD and sensitize cancer cells.

### SUMMARY

The present invention solves one or more problems of the prior art by providing, in at least one embodiment, a method for treating hyperglycemia or reducing glycemia in a subject undergoing chemotherapy or other cancer therapy. The method includes a step of identifying a subject undergoing chemotherapy and being administered a hyperglycemia-inducing agent. Short-term starvation, a fasting mimicking diet, or insulin are administered for a first time period to the subject to prevent or reverse hyperglycemia and sensitization to chemotherapy associated with increased glucose levels.

Various embodiments of the invention, alleviate or treat symptoms of chemotherapy which can be worsened by the complementary administration of rapamycin and the steroid medication dexamethasone. It is shown below (Figure 1B-C) that the administration of dexamethasone and rapamycin (**Figure 1B**, D) for the treatment of chemotherapy-associated side can cause sensitization of animals to chemotherapy. As shown below (Figure 1B-D), the administration of insulin to reduce circulating glucose levels in control mice, as well as in animals undergoing Rapa and Dexa treatment, can reverse the toxicity of doxorubicin and of other chemotherapy drugs. Because of the wide use of rapamycin and dexamethasone for the treatment of certain tumors in humans, these results have important implications for the safety of patients and efficacy of those therapies.

Because of its effects in reducing circulating glucose levels (**Figure 1E**) and up-regulating AMPK, which we have shown to inactivate PKA signaling, Metformin has the potential to be used as a STS-mimicking drug to (i) reverse the hyperglycemia-associated cytotoxic effects of chemotherapy and, when administered during the re-feeding period by both acting on glucose levels and PKA, to (ii) potentiate/prolong the effect of STS in reducing the tumor-progression, again by acting on both glucose and AMPK-PKA signaling. Thus, metformin can promote both differential stress resistance and differential stress sensitization by both reducing glucose levels and acting on PKA signaling as described in Raffaghello et.al., PNAS. 2008; PMID: 18378900 and Lee et.al. Sci Transl Med. 2012; PMID: 22323820.

In another embodiment, a method of replacing or enhancing effects of a fasting mimicking diet (FMD) on cancer cell sensitization is provided. The method includes a step of identifying a subject receiving chemotherapy or another cancer therapy. Metformin is then administered to the subject by administering to the subject.

In another embodiment, a method of promoting differential stress is provided. The method includes a step of identifying a subject with one or more of breast cancer, ovarian cancer, colorectal cancer, melanoma, prostate cancer, cervical cancer, epidermoid carcinoma, neuroblastoma, or any additional cancer type. Metformin is administered to the subject to reduce glucose levels and promote differential stress sensitization to specifically kill cancer but not normal cells.

### BRIEF DESCRIPTION OF THE DRAWINGS

**FIGURE 1A, 1B, 1C, 1D and 1E****.** Increase in circulating glucose levels mediates the sensitization of the host to chemotherapy. Administration of rapamycin (Rapa) or dexamathasone (Dexa) (**A**) caused an increase in glucose levels that was significantly reduced by insulin and, even more, by STS (**B**). Asterisks in **B** indicate the significance of each group compared to the ad lib (AL) group. The significance of each group compared to its internal control is indicated with daggers (i.e. the daggers on Rapa + ins and Dexa + ins indicate the significance compared to AL + ins). For the stress resistance experiment shown in **C** and **D** we followed the schedule shown in **A.** Rapamycin and Dexamethasone where administrated ip for 14 days prior DXR injection (day 0). Following the administration of 24 mg/kg of DXR, the animals where monitored for signs of distress and the survival was recorded (**C** and **D**). STS, ad lib, and ad lib + ins groups reported in **C** and **D** were shared groups and have the same values in both graphs. (**E**) Blood glucose levels in mice injected ip with metformin- 50 mg/kg (saline for control mice). One-way ANOVA test was performed and differences with p-value<0.05 were considered significant (p-value<0.05, 0.01 and 0.001 are indicated as *, *, and ***, respectively).

**FIGURE 2****.** Effect of glucose restriction on DXR sensitivity of 9 different mouse and human cancer cell lines. Control groups were cultured in DMEM supplemented with 2.0 g/L glucose, while the glucose restriction groups were cultured in DMEM supplemented with 0.5 g/L glucose. Survival was determined by MTT reduction. The cancer cell line tested were: 4T1 (mouse breast cancer), B16 (mouse melanoma), GL26 (mouse glioma), C42B (human prostate cancer), MCF-7 (human breast cancer), HeLa (human cervical cancer), A431 (human epidermoid carcinoma), ACN (human neuroblastoma), and MZ2-MEL (human melanoma).

**TABLE 1.** Micronutrient content provided by the Fasting Mimicking Diet (FMD)

### DETAILED DESCRIPTION

Reference will now be made in detail to presently preferred compositions, embodiments and methods of the present invention which constitute the best modes of practicing the invention presently known to the inventors. The Figures are not necessarily to scale. However, it is to be understood that the disclosed embodiments are merely exemplary of the invention that may be embodied in various and alternative forms. Therefore, specific details disclosed herein are not to be interpreted as limiting, but merely as a representative basis for any aspect of the invention and/or as a representative basis for teaching one skilled in the art to variously employ the present invention.

Except in the examples, or where otherwise expressly indicated, all numerical quantities in this description indicating amounts of material or conditions of reaction and/or use are to be understood as modified by the word "about" in describing the broadest scope of the invention. Practice within the numerical limits stated is generally preferred. Also, unless expressly stated to the contrary: percent, "parts of," and ratio values are by weight; the description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description and does not necessarily preclude chemical interactions among the constituents of a mixture once mixed; the first definition of an acronym or other abbreviation applies to all subsequent uses herein of the same abbreviation and applies *mutatis mutandis* to normal grammatical variations of the initially defined abbreviation; and, unless expressly stated to the contrary, measurement of a property is determined by the same technique as previously or later referenced for the same property.

It is also to be understood that this invention is not limited to the specific embodiments and methods described below, as specific components and/or conditions may, of course, vary. Furthermore, the terminology used herein is used only for the purpose of describing particular embodiments of the present invention and is not intended to be limiting in any way.

It must also be noted that, as used in the specification and the appended claims, the singular form "a," "an," and "the" comprise plural referents unless the context clearly indicates otherwise. For example, reference to a component in the singular is intended to comprise a plurality of components.

Throughout this application, where publications are referenced, the disclosures of these publications in their entireties are hereby incorporated by reference into this application to more fully describe the state of the art to which this invention pertains.

Abbreviations:
"AL" mean *ad lib.*
"FMD" means fasting mimicking diet.
"STS" means short-term starvation.
"DSR" means differential stress resistance.
"DSS" means differential stress Sensitization.
"DXR" means doxorubicin.
"Rapa" means rapamycin.
"Dexa" means dexamethasone.
"ins" means insulin.
"AMPK" means 5' AMP-activated protein kinase.
"ip" means intraperitoneal.

The terms "kilocalorie" (kcal) and "Calorie" refer to the food calorie.

The term "calorie" refers to the so-called small calorie.

The term "subject" refers to a human or animal, including all mammals such as primates (particularly higher primates), sheep, dog, rodents (e.g., mouse or rat), guinea pig, goat, pig, cat, rabbit, and cow.

The term "fasting mimicking diet" means a diet that provides the subject with a calorie restricted diets formulated in a way to generate changes in glucose, ketone bodies, IGF-1 and IGFBP1 similar to those caused by fasting but able to provide high nourishment and minimize hunger.

In an embodiment, methods for treating hyperglycemia in a subject undergoing chemotherapy are provided. The method includes a step of identifying a subject undergoing chemotherapy and being administered a hyperglycemia-inducing agent. Short-term starvation, a fasting mimicking diet (FMD) or insulin are administered for a first time period to the subject to prevent or reverse hyperglycemia and sensitization to chemotherapy associated with increased glucose levels. In the context of the present embodiment, preventing hyperglycemia or sensitization means reducing the probability that these side effect will occur. In general, the FMD diet provides less than about 1000 kilocalories per day, while STS provides no calories when administered. In a refinement, the hyperglycemia-inducing agent is a kinase inhibitor or a corticosteroid. Specific examples of hyperglycemia-inducing agent include, rapamycin, steroid medications including dexamethasone, and the like, and combinations thereof. In a refinement, short-term starvation or a fasting mimicking diet is repeated a plurality of times at predetermined intervals. For example, short-term starvation or a fasting mimicking diet can be repeated at intervals from two weeks to 2 months. Typically, he subject is administered a normal diet i.e., re-feeding period) in between these repetitions. In this context, a normal diet is a diet of sufficient caloric intake to maintain the patient weight. In a refinement, the normal caloric intake provides the subject with 1500 to 2500 kcal or 1800 to 2300 kcal, or 1800 to 2000 kcal.

Examples of STS protocols are found in U.S. Pat. Appl. Nos. 12/430,058 and 13/488,590; the entire disclosures of which are hereby incorporated by reference. In a variation, the STS diet provides a hypo-caloric or calorie free diet. The diet contains dietary materials capable of providing nutrition to a human subject while providing no more than 813-957 kcal (e.g., no more than 700, 500, 300, or 100 kcal, or 0 kcal) total energy, and no more than 30-36 g (e.g., no more than 20, 10, or 5 g, or 0 g) protein. If carbohydrates are present in the dietary materials, no more than half of the energy is in the carbohydrates. In a refinement, the STS/FMD diet may be administered to the subject for 3-10 consecutive days prior to when the subject is exposed to chemotherapy. The diet may also be administered to the subject for 24 hours following the exposure. Preferably, the diet may be administered to the subject for both 3-10 consecutive days prior to when the subject is exposed to chemotherapy and 24 hours following the exposure.

In another variation, the STS diet provides nutrition while providing no more than 11 kcal (e.g., no more than 8, 5, or 2 kcal, or 0 kcal) energy per kg body weight of the subject per day and no more than 0.4 g (e.g., 0.3, 0.2, or 0.1 g or 0 g) protein per kg body weight of the animal or human per day. If carbohydrates are present in the diet, no more than half of the energy is in the carbohydrates. In some embodiments, the diet is capable of providing no more than 700 kcal (e.g., 600, 400, or 200 kcal or 0 kcal) total energy per day. When the subject is exposed to chemotherapy, normal cells, but not abnormal cells such as cancer cells, in the animal or human are protected. For example, the diet may be administered to the animal or human for 3-10 consecutive days prior to the subject's exposure to chemotherapy. The diet may also be administered to the subject for 24 hours following the exposure. Preferably, the diet may be administered to the subject for both 3-10 consecutive days prior to the subject's exposure to chemotherapy and 24 hours following the exposure.

In another variation, the STS/FMD protocol involves fasting mimicking diets. For example, the subject suffering from cancer may be fasted for 48-140 hours prior to one round of chemotherapy or 4-56 hours following the chemotherapy. Preferably, the subject suffering from cancer is given a FMD for 48-140 hours prior to one round of chemotherapy and 4-56 hours following the chemotherapy. Examples of FMD diets are found in U.S. Pat. Appl. Nos. 14060494 and 14178953 and WIPO Pub. No. WO2011/050302 and WIPO Pub. No. WO2011/050302; the entire disclosures of which are hereby incorporated by reference. Typically, in the FMD protocol a subject's diet is substituted for a predetermined number of days (i.e., 5 days). During this period, subjects consume plenty of water. For healthy subjects of normal weight (Body Mass Index or BMI between 18.5-25), the diet is consumed once a month (5 days on the diet and 25-26 days on their normal diet) for the first 3 months and every 3 months thereafter (5 days every 3 months). The weight of the subject is measured and the subject must regain at least 95% of the weight lost during the diet before the next cycle is begun. Subjects with BMI of less than 18.5 should not undertake the FMD unless recommended and supervised by a physician. The same regimen (once every month for 3 months followed by once every 3 months thereafter) can be adopted for the treatment, or in support of the treatment, of all of the conditions presented in the patent applications. U.S. Pat. Appl. No. 14178953 provides a low protein version of the FMD diet.

In one variation, the FMD set forth in U.S. Pat. Appl. Nos. 12/430,058 is used in the methods set forth above. This diet includes nutrition facts relative to calories, macronutrients and micronutrients. Calories are consumed according to the user's body weight. Total calorie consumption is 4.5-7 calorie per pound (or 10-16 calorie per kilogram) for day 1 and 3-5 calorie per pound (or 7-11 calorie per kilogram) for day 2 to 5. Figures 12-14 provides listings of the nutrients for day one through day five. In addition to the macronutrients, the diet should contain less than 30 g of sugar on day 1 and less than 20 g of sugar on days 2-5. The diet should contain less than 28 g of proteins on day 1 and less than 18 g of proteins on days 2-5. The diet should contain between 20 and 30 grams of monounsaturated fats on day 1 and 10-15 grams of monounsaturated fats on days 2-5. The diet should contain between 6 and 10 grams of polyunsaturated fats on day 1 and 3-5 grams of polyunsaturated fats on days 2-5. The diet should contain less than 12 g of saturated fats on day 1 and less than 6 grams of saturated fats on days 2-5. Typically, the fats on all days are derived from a combination of the following: Almonds, Macadamia Nuts, Pecans, Coconut, Coconut oil, Olive Oil and Flaxseed. In a refinement, the FMD diet includes over 50% of the recommended daily value of dietary fiber on all days. In the further refinement, the amount of dietary fiber is greater than 15 grams per day on all five days. The diet should contain 12-25 grams of glycerol per day on days 2-5. In a refinement, glycerol is provided at 0.1 grams per pound body weight/day.

In a refinement, the FMD includes the following micronutrients (at least 95% non-animal based): over 5,000 IU of vitamin A per day (days 1-5); 60-240 mg of vitamin C per day (days 1-5); 400-800 mg of Calcium per day (days 1-5); 7.2-14.4 mg of Iron per day (days 1-5); 200-400 mg of Magnesium per day (days 1-5); 1-2 mg of copper per day (days 1-5); 1-2 mg of Manganese per day (days 1-5); 3.5-7 mcg of Selenium per day (days 1-5); 2-4 mg of Vitamin B1 per day (days 1-5); 2-4 mg of Vitamin B2 per day (days 1-5); 20-30 mg of Vitamin B3 per day (days 1-5); 1-1.5 mg of Vitamin B5 per day (days 1-5); 2-4 mg of Vitamin B6 per day (days 1-5); 240-480 mcg of Vitamin B9 per day (days 1-5); 600-1000 IU of Vitamin D per day (days 1-5); 14-30 mg of Vitamin E per day (days 1-5); over 80 mcg of Vitamin K per day (days 1-5); 16-25 mcg Vitamin B12 are provided during the entire 5-day period; 600 mg of Docosahexaenoic acid (DHA, algae-derived) are provided during the entire 5-day period. The FMD diet provides high micronutrient content mostly (i.e., greater than 50 percent by weight) from natural sources including: Kale, Cashews, Yellow Bell Pepper, Onion, Lemon Juice, Yeast, Turmeric. Mushroom, Carrot, Olive Oil, Beet Juice, Spinach, Tomato, Collard, Nettle, Thyme, Salt, Pepper, Vitamin B12 (Cyanocobalamin), Beets, Butternut Squash, Collard, Tomato, Oregano, Tomato Juice, Orange Juice, Celery, Romaine Lettuce, Spinach, Cumin, Orange Rind, Citric Acid, Nutmeg, Cloves, and combinations thereof. Table 1 provides an example of additional micronutrient supplementation that can be provided in the FMD diet:

**Table 1. Micronutrient Supplementation**

| | **Supplement** | **Formula** | **Amount** | **Amount Range** | **Unit** |
|---|---|---|---|---|---|
| **Vit A** | | | 1250 IU | 900-1600 | IU |
| **Vit C** | Ascorbic Acid | C₆H₈O₆ | *15.0000* | 10-20 | mg |
| **Ca** | Calcium Carbonate | CaCO₃ | *80.0000* | 60-100 | mg |
| **Fe** | Ferrous Fumarate | C₄H₂FeO₄ | *4.5000* | 3-6 | mg |
| **Vit** D3 | Cholecalciferol | C₂₇H₄₄O | *0.0025* | 0.001-0.005 | mg |
| **VitE** | dl-Alpha Tocopheryl Acetate | C₂₉H₅₀O₂ | *5.0000* | 3-7 | mg |
| **Vit K** | Phytonadione | | *0.0200* | 0.1-0.04 | mg |
| **Vit B1** | Thiamine Mononitrate | C₁₂H₁₇N₅O₄S | *0.3750* | 0.15-0.5 | mg |
| **Vit B2** | Riboflavin E101 | C₁₇H₂₀N₄O₆ | *0.4250* | 0.2-0.6 | mg |
| **Vit B3** | Niacinamide | C₆H₆N₂O | *5.0000* | 3-7 | mg |
| **Vit B5** | Calcium Pantothenate | C₁₈H₃₂CaN₂O₁₀ | *2.5000* | 1.5-4.0 | mg |
| **Vit B6** | Pyridoxine Hydrochloride | C₈H₁₁NO₃ · HCl | *0.5000* | 0.3-0.7 | mg |
| **Vit B7** | Biotin | C₁₀H₁₆N₂O₃S | *0.0150* | 0.01-0.02 | mg |
| **Vit B9** | Folic Acid | C₁₉H₁₉N₇O₆ | *0.1000* | 0.07-0.14 | mg |
| **Vit B12** | Cyanocobalamin | C₆₃H₈₈CoN₁₄O₁₄P | *0.0015* | 0.001-0.002 | mg |
| **Cr** | Chromium Picolinate | Cr(C6H4NO2)3 | *0.0174* | 0.014-0.022 | mg |
| **Cu** | Cupric Sulfate | CuSO4 | *0.2500* | 0.18-0.32 | mg |
| **I** | Potassium Iodide | KI | *0.0375* | 0.03-0.045 | mg |
| **Mg** | Magnesium Oxide | MgO | *26.0000* | 20-32 | mg |
| **Mn** | Manganese Sulfate | MnSO₄ | *0.5000* | 0.3-0.7 | mg |
| **Mo** | Sodium Molybdate | Na₂MoO₄ | *0.0188* | 0.014-0.023 | mg |
| **Se** | Sodium Selenate | Na₂O₄Se | *0.0175* | 0.014-0.023 | mg |
| **Zn** | Zinc Oxide | **ZnO** | *3.7500* | 3-5 | mg |

In another embodiment, a diet package for implemented the method forth above is provided. The diet package includes a first set of rations for a first diet to be administered for a first time period to a subject, the first diet providing from 4.5 to 7 kilocalories per pound of subject for a first day and 3 to 5 kilocalories per pound of subject per day for a second to fifth day of the first diet. The diet package includes rations that provide less than 30 g of sugar on the first day; less than 20 g of sugar on the second to fifth days; less than 28 g of proteins on the first day; less than 18 g of proteins on days the second to fifth days; 20 to 30 grams of monounsaturated fats on the first day; 10 to 15 grams of monounsaturated fats on the second to fifth days; between 6 and 10 grams of polyunsaturated fats on the first day; 3 to 5 grams of polyunsaturated fats on the second to fifth days; less than 12 g of saturated fats on the first day; less than 6 grams of saturated fats on the second to fifth days; and 12 to 25 grams of glycerol per day on the second to fifth days. In a refinement, the diet package further includes sufficient rations to provide the micronutrients set forth above. In a further refinement, the diet package provides instructions providing details of the methods set forth above.

In refinement of the embodiments set forth above, a 5-day supply of diet includes: soups/broths, soft drinks, nut bars and supplements. The diet is administered as follows: 1) on the first day a 1000-1200 kcal diet with high micronutrient nourishment as set forth above is provided; 2) for the next 4 days a daily diet of 650- 800 kcal plus a drink containing a glucose substitution carbon source providing between 60-120 kcal are provided.

In another refinement of the embodiments set forth above, a 6-day low-protein diet protocol includes: soups/broths, soft drinks, nut bars, and supplements. The diet is administered as follows: 1) on the first day a 1000-1200 kcal diet plus with high micronutrient nourishment is provided; 2) for the next 3 days a daily diet of less than 200 kcal plus a drink containing a glucose substitution carbon source providing between 60 and 120 kcal. This substitution carbon source does not interfere with the effect of fasting on stem cell activation; 3) on the 5th day the subject consumes a normal diet; and 4) on day 6 an additional replenishment foods consisting of a high fat source of 300 kcal and a micronutrient nourishment mix on day 6 replenishment foods consisting of a high fat source of 300 kcal and a micronutrient nourishment mix are provided in addition to normal diet.

In still another refinement, a diet protocol includes: 6-day supply of low-protein diet includes: soups/broths, soft drinks, nut bars, and supplements. 1) on the first day a 1000-1200 kcal diet with high micronutrient nourishment is provided; 2) for the next 3 days a daily diet of 600 to 800 kcal which contains less than 10 grams of protein and less than 200 kcal from sugars; 3) on the 5th day the subject receives a normal diet; and 4 ) on day 6 an additional replenishment foods consisting of a high fat source of 300 kcal and a micronutrient nourishment mix on day 6 replenishment foods consisting of a high fat source of 300 kcal and a micronutrient nourishment mix are provided in addition to normal diet.

Although the FMD diet encompasses virtually any source of fat, sources high in unsaturated fat, including monounsaturated and polyunsaturated fat sources, are particularly useful (e.g., omega-3/6 essential fatty acids). Suitable examples of monounsaturated food sources include, but are not limited to, peanut butter, olives, nuts (e.g., almonds, pecans, pistachios, cashews), avocado, seeds (e.g., sesame), oils (e.g., olive, sesame, peanut, canola), etc. Suitable examples of polyunsaturated food sources include, but are not limited to, walnuts, seeds (e.g., pumpkin, sunflower), flaxseed, fish (e.g., salmon, tuna, mackerel), oils (e.g., safflower, soybean, corn). The first diet also includes a component selected from the group consisting of vegetable extracts, minerals, omega-3/6 essential fatty acids, and combinations thereof. In one refinement, such a vegetable extract provides the equivalent of 5 recommended daily servings of vegetables. Suitable sources for the vegetable extract include, but are not limited to, bokchoy, kale, lettuce, asparagus, carrot, butternut squash, alfalfa, green peas, tomato, cabbage, cauliflower, beets. Suitable sources for the omega-3/6 essential fatty acids include fish such as salmon, tuna, mackerel, bluefish, swordfish, and the like.

In another variation, a method based on the administration of Metformin (*N,N-*Dimethylimidodicarbonimidic diamide) to mimic the effects of fasting to reverse the hyperglycemia-associated cytotoxic effects of chemotherapy and/or to potentiate/prolong the effect of STS in reducing the tumor-progression when administered during the re-feeding period is provided. The method includes a step of identifying a subject undergoing chemotherapy and having hyperglycemia and/or being administered a hyperglycemia-inducing agent as set forth above. Metformin is administered to the subject to reverse the cytotoxic effects. Metformin is administered in a dosage range from 1 to 2.5 mg/day depending on the response of the patient to the drug. The Metformin can be administered for 1 day, 1 to 5 days, 1 to 10 days, or 1 to 14 days or more depending on the subject's response. The In another variation, a method for treating hyperglycemia or the negative effects of normo-glycemia in a subject undergoing chemotherapy or another cancer therapy is provided. The method includes a step of identifying a subject undergoing chemotherapy and being administered a hyperglycemia-inducing agent. Short-term starvation or a fasting mimicking diet, or insulin is administered for a first time period to the subject to prevent or reduce glucose levels and sensitize cancer cells to chemotherapy or other cancer therapy. The details of this variation regarding the administration of short-term starvation or a fasting mimicking diet are the same as those set forth above. During a re-feeding period, a normal diet is administered to the subject in between administration of the short-term starvation or a fasting mimicking diet also as set forth above. Metformin is administered to the subject during this re-feeding period. Metformin is administered in a dosage range from 1 to 2.5 mg/day depending on the response of the patient to the drug. The Metformin can be administered for 1 day, 1 to 5 days, 1 to 10 days, or 1 to 14 days or more depending on the subject's response. The steps of administration of short-term starvation or a fasting mimicking diet and administering the re-feeding period with Metformin administration is repeated is repeated a plurality of times at predetermined intervals. As set forth above, in a refinement, these steps are repeated at intervals from two weeks to 2 months.

In another embodiment, a method of replacing or enhancing the effect of the FMD on cancer cell sensitization is provided. The method includes a step of identifying a subject receiving chemotherapy or another cancer therapy. Metformin is then administered to the subject by administering to the subject. In a refinement, Metformin is administered in a dosage range from 1 to 2.5 mg/day depending on the response of the patient to the drug. The Metformin can be administered for 1 day, 1 to 5 days, 1 to 10 days, 1 to 14 days or 1 to 60 days or more depending on the subject's response.

In another embodiment, a method of promoting differential stress is provided. The method includes a step of identifying a subject with one or more of breast cancer, ovarian cancer, colorectal cancer, melanoma, prostate cancer, cervical cancer, epidermoid carcinoma, neuroblastoma, or any additional cancer type. Metformin is administered to the subject to reduce glucose levels and promote differential stress sensitization to specifically kill cancer but not normal cells. Metformin is administered in a dosage range from 1 to 2.5 mg/day depending on the response of the patient to the drug. The Metformin can be administered for 1 day, 1 to 5 days, 1 to 10 days, 1 to 14 days, or 1 to 60 days or more depending on the subject's response.

The following examples are intended to illustrate, but not to limit, the scope of the invention. While such examples are typical of those that might be used, other procedures known to those skilled in the art may alternatively be utilized. Indeed, those of ordinary skill in the art can readily envision and produce further embodiments, based on the teachings herein, without undue experimentation.

The present invention has been tested in *in vitro* and *in vivo* murine models. STS and FMD have also been tested in different clinical trials which have shown the safety and feasibility of the two dietary interventions. FMD diet has shown to be as effective as STS in evoking DSR.

Methods: Rapamycin or Dexamethasone were daily administrated intraperitoneally (ip) for a period of 14 days prior the beginning of Short term starvation (STS) or fasting mimicking diet (FMD). The end of the dietary intervention coincided with the administration of doxorubicin by intravenous injection. Mice in the insulin (ins) groups also received insulin injection every 12h for the 48h preceding doxorubicin administration. The animals were then being observed for sign of pain or distress for the following days and the survival was recorded (Figure 1A, C, and D).

Metformin (50 mg/kg) was diluted in saline and administrated by intraperitoneal (i.p.) injection. Circulating glucose levels were monitored following metformin administration (Figure 1E).

Diet (mouse): Mice were maintained on irradiated TD.7912 rodent chow (Harlan Teklad). ln brief, this diet contains 3.T5kcal/g of digestible energy with calories supplied by protein, carbohydrate and fat in a percent ratio of25: 58: 17. Food was provided ad lib. On average, mice in the control group consumed 14.9 kcal/day (or 3.9 g/day), Our experimental FMD diet is based on a nutritional screen that identified ingredients allowing high nourishment during periods of low calorie consumption (Brandhorst, Wei et al., 2013). Prior to supplying the FMD diet, animals were transferred into fresh cages to avoid feeding on residual chow and coprophagy. The FMD diet consists of two different components designated as day 1 diet and day 2-4 diet that were fed in this order, respectively. The day 1 diet contains 1.88 kcal/g and was designed to adapt the mouse to a period of low caloric intake during the subsequent feeding days. The day 2-4 diet is identical on all feeding days and contains 0.36 kcal/g. The day 1 and days 2-4 diets were fed as the average intake (~4 g) of the *ad lib* fed control group every two weeks. Due to the different caloric densities of the supplied FMD diet, mice in this cohort had a ~50% reduction in consumed calories on day 1 and consumed 9.7% of the control cohort on days 2 to 4. Mice consumed all the supplied food on each day of the FMD regimen and showed no signs of food aversion. After the end of the day 2-4 diet, we supplied TD.7912 chow *ad lib* for 10 days before starting another FMD cycle.

Diet (Human): The FMD will substitute the normal diet of a cancer patient for a period of 5 to 21 days with a 17 day maximum for most patients (see below) with frequency to be determined based on the frequency and efficacy of other treatments, with more frequent use needed when other treatments are not effective in cancer treatment. The ability of the patient to regain weight before the next cycle is initiated must also be considered, with patients with more severe symptoms able to regain weight receiving the diet as frequently as the other treatments are given and patients who are not regaining weight or are unable to undergo the full dietary period being placed on the FMD only after they return to the normal weight (weight before treatment is initiated but also BMI above 18). The FMD consists of ingredients which are Generally Regarded As Safe (RGAS). Calories are consumed according to the subject's body weight. For day 1, total calorie consumption is 4.5-7 calorie per pound (or 10-16 calorie per kilogram). The diet should be at least 90% plant based. The day 1 diet should contain less than 30 g of sugars, less than 28 g of plant based proteins, 20-30 grams of plant based monounsaturated fats, 6-10 g of plant based polyunsaturated fats and 2-12 g of plant based saturated fats. For days 2-21, total calorie consumption is 3-5 calorie per pound (or 7-11 calorie per kilogram). The days 2-21 diet should contain less than 20 g of sugars, less than 18 g of plant based proteins, 10-15 g of plant based monounsaturated fats, 3-5 g of plant based polyunsaturated fats and 1-6 grams of plant based saturated fats, 10-30 grams of glycerol diluted in 1 liter of water/day, based on body weight (10 grams for a 100 pound person, 20 grams for a 200 pound person and 30 grams for a 300 pound person). Diet should also be high nourishment containing approximately 50% of the RDA (daily) for vitamins, minerals + essential fatty acids. The minimum length will be 5 or 6 days and the maximum length 2l days (based on safety data and standard of care practice at fasting clinics).

*In vitro* dose response of cancer cell lines to DXR: glucose restriction was applied to cells 24 hours before and 24 hours during DXR treatment. Control groups were cultured in DMEM supplemented with 2.0 g/L glucose while the glucose restriction groups were cultured in DMEM supplemented with 0.5 g/L glucose. Survival was determined by MTT reduction.

Stress resistance - 12 weeks old female C57BL/6 mice were divided in the following experimental groups; *ad lib* (*ad libitum* feeding), STS/FMD, DXR, STS/FMD + DXR. In order to observe the response to every treatment in presence or not of rapamycin and dexamethasone, each group was present as triplicate where one of the sets underwent rapamycin treatment and one underwent dexamethasone treatment. The administration of rapamycin was performed for a period of 14 days at the end of which a high dose of doxorubicin was administrated iv (24 mg/kg/mouse). The administration of dexamethasone was performed for a period of 14 days at the end of which a high dose of doxorubicin was administrated iv (24 mg/kg/mouse). The animals belonging to the STS + DXR groups were fed a very low calorie and no protein FMD for 48h prior the injection of doxorubicin. Following doxorubicin injection the animals were monitored every day and the survival was recorded (Figure 1A, B, and C). Mice in the insulin (ins) groups also received insulin injection every 12h for the 48h preceding doxorubicin administration.

It is observed that the administration of the kinase inhibitor rapamycin, corticosteroid drugs such as dexamethasone and of other hyperglycemia-inducing drugs during chemotherapy sensitizes mice to the drug leading to an increased mortality (**Figure 1C and 1D**). In addition, the sensitization of the animals is positively associated with an increase of circulating blood glucose (**Figure 1B**). However, when the glucose levels are reduced by either STS, FMD, or the administration of insulin, this sensitizing effect is completely or partially reversed, respectively (Figure 1B-D). The experiments show that administration of insulin, STS, or FMD in combination with rapamycin and dexamethasone: a) offer a powerful tool to reduce the hyperglycemic state induced by rapamycin and dexamethasone (**Figure 1B**) and b) to reverse the toxic effects associated with the hyperglycemia induced by the two drugs (**Figure 1C and 1D**).

While exemplary embodiments are described above, it is not intended that these embodiments describe all possible forms of the invention. Rather, the words used in the specification are words of description rather than limitation, and it is understood that various changes may be made without departing from the spirit and scope of the invention. Additionally, the features of various implementing embodiments may be combined to form further embodiments of the invention.

## Claims

1. A method for treating a hyperglycemia or normo-glycemia in a subject undergoing chemotherapy, the method comprising:
a) identifying a subject undergoing chemotherapy and being administered a hyperglycemia-inducing agent; and
b) administering a fasting mimicking diet FMD to the subject for a first time period to reduce glucose levels and to sensitize cancer cells to chemotherapy; and
c) administering a normal diet to the subject during a re-feeding period; and
d) administering metformin to the subject during the re-feeding period.

2. The method of claim 1 wherein the hyperglycemia-inducing agent is selected from the group consisting of rapamycin, steroid medications including dexamethasone, and combinations thereof.

3. The method of claim 1 wherein the FMD is administered for 48-140 hours prior to a round of chemotherapy and/or 4-56 hours following a round of chemotherapy.

4. The method of claim 1 wherein fasting mimicking diet FMD is administered repeatedly a plurality of times at predetermined intervals.

5. The method of claim 4 wherein fasting mimicking diet FMD is administered repeatedly at intervals from two weeks to 2 months.

6. The method of claim 5 wherein the subject is administered a normal diet in between repetition of the FMD.

7. The method of claim 1 wherein the first time period is from 3 to 10 days.

8. The method of claim 1 wherein the hyperglycemia-inducing agent is rapamycin.

9. A method for treating a hyperglycemia in a subject undergoing chemotherapy, the method comprising:
a) identifying a subject undergoing chemotherapy and having hyperglycemia and/or being administered a hyperglycemia-inducing agent; and
b) administering metformin to subject to mimic effects of fasting.

10. The method of claim 9 wherein the hyperglycemia-inducing agent is selected form the group consisting of rapamycin, steroid medications including dexamethasone, and combinations thereof.

11. The method of claim 9 wherein the metformin is administered at a dose of 1 to 2.5 mg/day.

12. A method for treating a hyperglycemia in a subject undergoing chemotherapy or other cancer therapy, the method comprising:
a) identifying a subject undergoing chemotherapy and having hyperglycemia and/or being administered a hyperglycemia-inducing agent;
b) administering short-term starvation STS, a fasting mimicking diet FMD; or insulin to the subject for a first time period to prevent or reverse hyperglycemia and sensitization to chemotherapy associated with increased glucose levels; and
c) administering a normal diet to the subject after step b);
d) administering metformin to the subject during the normal diet.

13. The method of claim 12 wherein the hyperglycemia-inducing agent is selected form the group consisting of rapamycin, steroid medications including dexamethasone, and combinations thereof.

14. The method of claim 12 wherein steps b) and c) are repeated a plurality of times at predetermined intervals.

15. The method of claim 14 wherein steps b) and c) are repeated at intervals from two weeks to 2 months.

16. The method of claim 12 wherein the first time period is from 3 to 10 days.

17. The method of claim 12 wherein the metformin is administered at a dose of 1 to 2.5 mg/day.

18. A diet package for its use in the treatment of hyperglycemia or normo-glycemia in a subject undergoing chemotherapy,
wherein said diet package comprises a fasting mimicking diet FMD to be administered to the subject for a first period when the subject is undergoing chemotherapy and being administered a hyperglycemia-inducing agent, to reduce glucose levels and to sensitize cancer cells to chemotherapy; and
wherein said diet package comprising metformin administered during a re-feeding period with a normal diet.
